(19) Europäisches Patentamt
European Patent Office
Office européen des brevets

(11) EP 0 796 613 B1

(12) **FASCICULE DE BREVET EUROPEEN**

(45) Date de publication et mention de la délivrance du brevet:
**24.02.1999 Bulletin 1999/08**

(51) Int. Cl.$^6$: **A61K 7/42**

(21) Numéro de dépôt: **97400484.8**

(22) Date de dépôt: **03.03.1997**

# (54) Procédé de reduction du photobleuis sement d'une composition cosmétique contenant des pigments d'oxyde de titane

Verfahren zum Vermindern von Photoverblauung von einer kosmetischen Zusammensetzung die Titandioxidpigmente enthält

Process for reducing the photoblueing of a cosmetic composition containing titanium oxide pigments

(84) Etats contractants désignés:
**DE ES FR GB IT**

(30) Priorité: **22.03.1996 FR 9603623**

(43) Date de publication de la demande:
**24.09.1997 Bulletin 1997/39**

(73) Titulaire: **L'OREAL**
**75008 Paris (FR)**

(72) Inventeur: **Ascione, Jean-Marc**
**Hoboken, New Jersey 07030 (US)**

(74) Mandataire: **Miszputen, Laurent**
**L'OREAL**
**Département Propriété Industrielle**
**Centre Charles Zviak**
**90, rue du Général Roguet**
**92583 Clichy Cédex (FR)**

(56) Documents cités:
**EP-A- 0 742 003**
**FR-A- 2 642 968**
**FR-A- 2 680 684**
**FR-A- 2 695 560**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 796 613 B1

## Description

La présente invention concerne un procédé pour réduire le photobleuissement d'une composition contenant des pigments d'oxyde de titane, consistant à introduire dans ladite composition au moins une silicone benzotriazole spécifique. Elle concerne également de nouvelles compositions cosmétiques comprenant des pigments d'oxyde de titane et au moins une silicone benzotriazole spécifique.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Pour filtrer les rayonnements UV-A et UV-B, il existe sur le marché différents types de filtres solaires : les pigments minéraux et les filtres organiques. Ces filtres doivent pouvoir absorber ou bloquer les rayons nocifs du soleil tout en restant inoffensifs pour l'utilisateur.

A ce titre, les pigments d'oxydes métalliques sont de plus en plus utilisés dans les produits solaires et les produits de jour, notamment de maquillage, compte tenu de leurs propriétés de diffusion et de réflexion des rayonnements UV qui leur confèrent un grand intérêt en terme de photoprotection : utilisés seuls, ils permettent d'obtenir une bonne protection contre les rayons UV ; associés à des filtres organiques, ils permettent de réaliser des produits hautement photoprotecteurs.

Ainsi, le pigment minéral le plus courant utilisé à ce jour est l'oxyde de titane, de préférence sous forme nanopigmentaire, dont les propriétés filtrantes sont bien connues.

Cependant, on observe une instabilité à la lumière des compositions contenant des pigments d'oxyde de titane dans un milieu sans oxygène, instabilité qui se manifeste par l'apparition d'une coloration bleue. Cette photocoloration, connue sous le nom de photobleuissement, n'est évidemment pas souhaitable d'un point de vue esthétique.

En vue de limiter ce phénomène de photoréactivité, on a proposé des pigments de $TiO_2$ traités en surface : ainsi, le brevet EP-B-0 461 130 décrit des nanoparticules de $TiO_2$ traitées par des anions phosphate. De même, il est connu en cosmétique d'utiliser des pigments de $TiO_2$ traités en surface par de la silice ou de l'alumine. Cependant, ces traitements sont coûteux et difficiles à mettre en oeuvre.

Enfin, s'il est vrai que ces traitements de surface permettent de réduire la photo-instabilité des pigments d'oxydes métalliques, et en particulier le photobleuissement des formules contenant des pigments de $TiO_2$, cette diminution peut encore apparaître comme insuffisante.

Le besoin subsiste donc toujours quant à pouvoir disposer de compositions photostables contenant des pigments d'oxyde de titane. Au sens de la présente invention, on entend par photostable le fait qu'une composition donnée ne soit pas ou peu sujette au phénomène de photocoloration décrit ci-avant.

Ainsi, à la suite d'importantes recherches menées dans le domaine de la photoprotection évoqué ci-dessus, la Demanderesse a maintenant découvert que l'introduction, dans une composition contenant des pigments de $TiO_2$, d'au moins une silicone benzotriazole spécifique, réduisait significativement le phénomène de photobleuissement intrinsèquement attaché à cette composition.

Cette découverte est à la base de l'invention.

La présente invention a ainsi pour premier objet un procédé pour réduire le photobleuissement d'une composition contenant des pigments d'oxyde de titane, consistant à introduire dans la composition au moins une silicone benzotriazole répondant à l'une des formules suivantes :

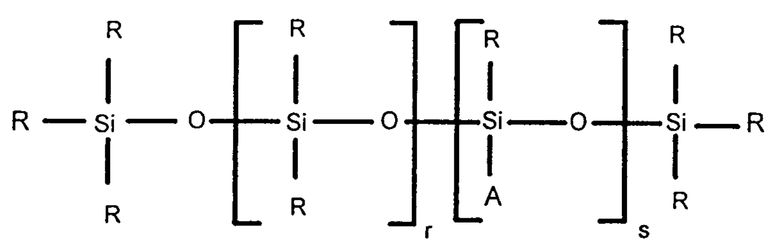

(1)

ou

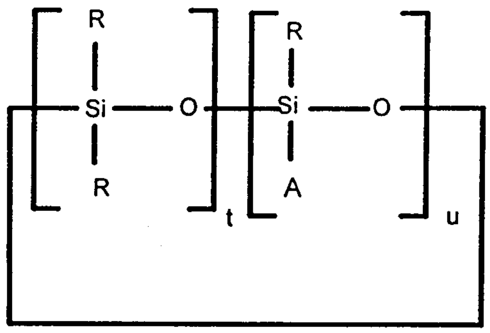

(2)

formules (1) et (2) dans lesquelles :

- R, identiques ou différents, sont choisis parmi les radicaux alkyles en $C_1$-$C_{10}$, phényle, trifluoro-3,3,3 propyle et triméthylsilyloxy, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

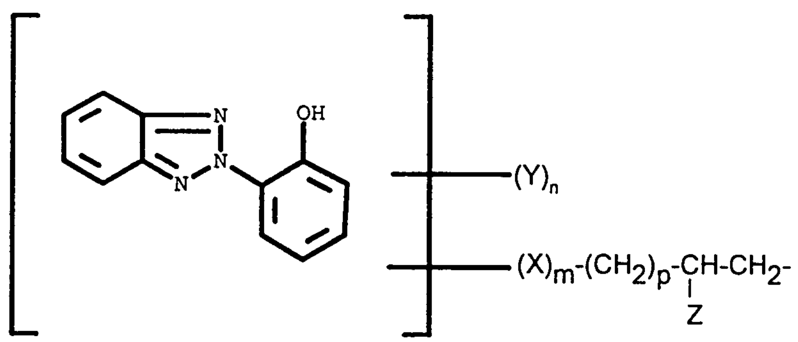

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

La présente invention a également pour objet l'utilisation d'au moins une silicone benzotriazole telle que définie ci-dessus dans ou pour la fabrication d'une composition cosmétique et/ou dermatologique contenant des pigments d'oxyde de titane, pour diminuer le photobleuissement dû à la présence desdits pigments dans ladite composition.

La présente invention a encore pour objet de nouvelles compositions cosmétiques et/ou dermatologiques, en particulier pour la photoprotection de la peau et/ou des cheveux, comprenant, dans un support cosmétiquement acceptable, des pigments d'oxyde de titane et au moins une silicone benzotriazole telle que définie ci-dessus, sous réserve

que cette composition est exempte d'acide benzène 1,4-di(3-méthylidène-10-camphosulfonique) et de 4,4'-méthoxy-t.-butyldibenzoyl-méthane.

En effet, il a été décrit dans la demande de brevet française n° 95-05677 (non encore publiée) déposée par la Demanderesse des compositions comprenant des silicones benzotriazoles conformes à celles de la présente invention en association avec l'acide benzène 1,4-di(3-méthylidène-10-camphosulfonique). Il est indiqué dans cette demande, d'une manière générale, que ces compositions peuvent également contenir des nanopigments d'oxyde de titane. Toutefois, l'effet de réduction du photobleuissement des pigments d'oxyde de titane apporté par la présence d'une silicone benzotriazole n'est absolument pas décrit ou enseigné par ce document.

Il a également été décrit dans l'exemple 2 de la demande FR-A-2 695 560 toujours au nom de la Demanderesse, une composition cosmétique filtrante comprenant des nanopigments d'oxyde de titane, une silicone benzotriazole conforme à celles de la présente invention et du 4,4'-méthoxy-t.-butyldibenzoylméthane. Selon l'invention de ladite demande, on vise à stabiliser du 4,4'-méthoxy-t.-butyldibenzoylméthane à l'aide d'un polymère filtre du type silicone benzotriazole. Toutefois, la réduction du photobleuissement des pigments d'oxyde de titane apportée par la présence d'une silicone benzotriazole n'est à non plus ni décrite ni enseignée.

Les compositions selon l'invention présentent l'avantage d'être très peu sujettes au phénomène de photobleuissement habituellement observé sur les compositions contenant des pigments d'oxyde de titane.

La présente invention a encore pour objet un procédé de traitement cosmétique pour protéger la peau et/ou les cheveux contre le rayonnement ultraviolet, en particulier le rayonnement solaire, consistant à appliquer sur ceux-ci une quantité efficace d'au moins une composition telle que définie ci-dessus.

D'autres caractéristiques, aspects et avantages de la présente invention apparaîtront à la lecture de la description détaillée qui va suivre.

Les silicones benzotriazoles spécifiques utilisées dans le cadre de la présente invention sont sélectionnées au sein de la famille générale connue des silicones benzotriazoles, et sont celles qui répondent aux formules suivantes:

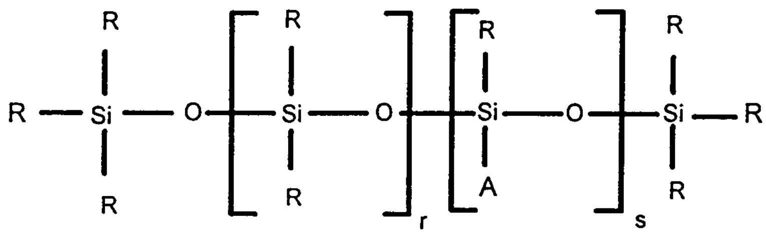

(1)

ou

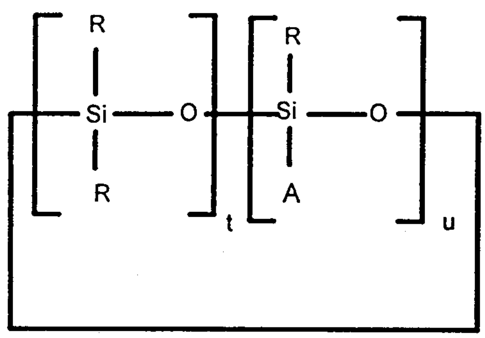

(2)

formules (1) et (2) dans lesquelles :

- R, identiques ou différents, sont choisis parmi les radicaux alkyles en $C_1$-$C_{10}$, phényle, trifluoro-3,3,3 propyle et triméthylsilyloxy, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,

- et le symbole A désigne un radical monovalent lié directement à un atome de silicium, et qui répond à la formule suivante :

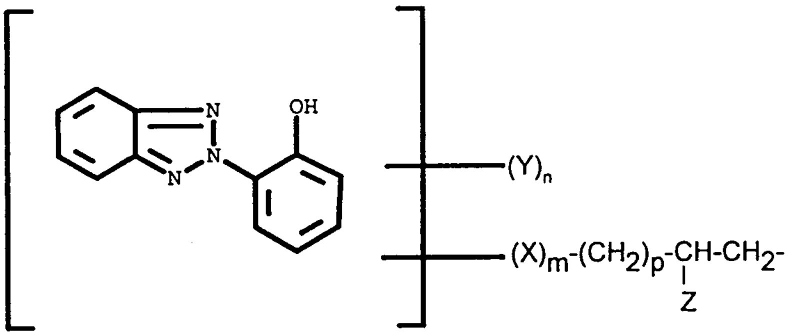

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Comme cela ressort de la formule (3) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

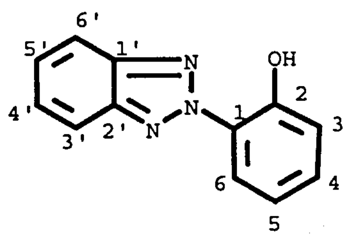

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif substituant Y se fait en position 5.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention sont les radicaux méthyle, éthyle, propyle, n-butyle, n-octyle et éthyl-2 hexyle. Encore plus préférentiellement, les radicaux R sont tous des radicaux méthyle.

Parmi les composés de formules (1) ou (2) ci-dessus, on préfère mettre en oeuvre ceux répondant à la formule (1), c'est-à-dire des diorganosiloxanes à chaîne courte linéaire.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

Une famille de composés convenant particulièrement à l'invention est celle définie par la formule générale (4) suivante :

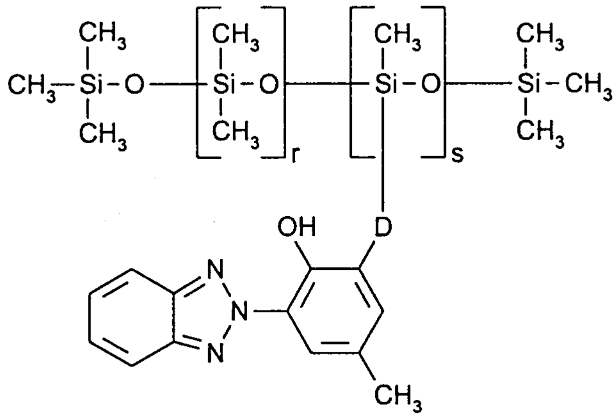

(4)

avec

$0 \leq r \leq 10$,
$1 \leq s \leq 10$,

et où D représente le radical divalent :

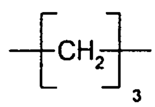

ou

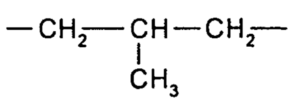

Dans une forme particulièrement préférée de réalisation de l'invention, la silicone benzotriazole est le composé (appelé composé (a) dans la suite du texte) répondant à la formule générale (4) dans laquelle :

r=0
s=1
D=

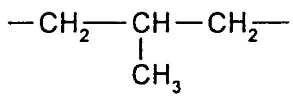

Dans une autre forme particulièrement préférée de réalisation de l'invention, la silicone benzotriazole est le composé (appelé composé (b) dans la suite du texte) répondant à la formule générale (4) dans laquelle :

r = 5
s = 5

et D représente le radical divalent :

$$-[CH_2]_3-$$

Pour préparer les filtres siliconés de formule (1) et (2), on peut procéder classiquement en mettant en oeuvre une réaction d'hydrosylilation (à savoir

$$\equiv Si\text{-}H + CH_2\text{=}C\text{-} \text{ -------->} \equiv Si\text{-}CH_2\text{-}CH\text{-})$$

à partir de la silicone correspondante dans laquelle, par exemple, tous les radicaux A sont des atomes d'hydrogène. Cette silicone de départ est dénommée par la suite dérivé à SiH. Ces dérivés à SiH sont des produits bien connus dans l'industrie des silicones et sont généralement disponibles dans le commerce. Ils sont par exemple décrits dans les brevets américains US-A- 3 220 972, US-A- 3 697 473 et US-A- 4 340 709. Ce dérivé à SiH peut être donc représenté soit par la formule (1bis) suivante :

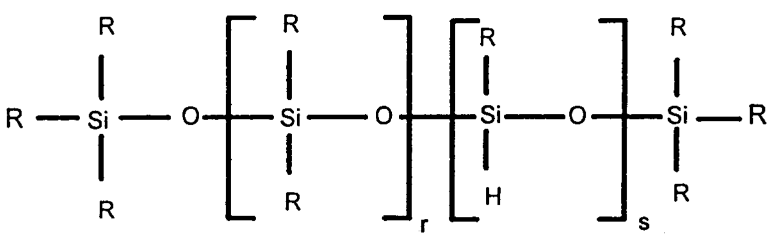

(1bis)

dans laquelle R, r et s ont la signification donnée ci-dessus pour la formule (1), soit par la formule (2bis) suivante :

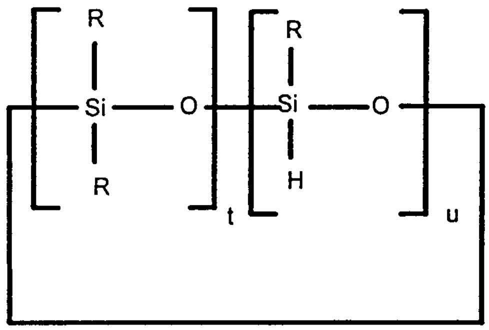

(2bis)

dans laquelle R, t et u ont la signification donnée ci-dessus pour la formule (2).

Sur ce dérivé à SiH de formules (1bis) ou (2bis), on effectue donc une réaction d'hydrosilylation classique, opérée en présence d'une quantité catalytiquement efficace d'un catalyseur au platine, sur un dérivé organique de benzotriazole de formule (3bis) suivante :

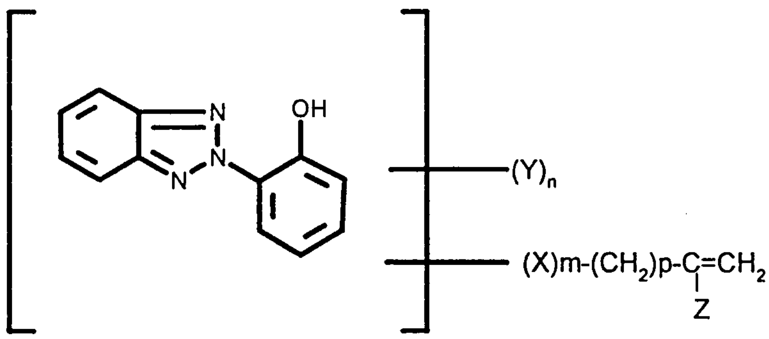

(3bis)

dans laquelle Y, X, Z, n, m et p ont la signification donnée ci-dessus pour la formule (3).

Des procédés convenant à la préparation des produits de formule (3bis) ci-dessus sont notamment décrits dans les brevets US- 4 316 033 et US- 4 328 346.

En outre, les détails des conditions opératoires à suivre pour conduire la réaction d'hydrosylilation entre les composés de formule (1bis) ou (2bis) ci-dessus avec le composé de formule (3bis) ci-dessus sont donnés dans la demande de brevet EP- 0 392 883, dont l'enseignement est, à cet égard, totalement inclus à titre de référence dans la présente description.

Dans les compositions de l'invention, on utilise généralement de 0,1 à 15 % en poids, de préférence de 0,2 à 10 % en poids, par rapport au poids total de la composition, d'une silicone benzotriazole telle que définie ci-dessus.

Une des caractéristiques essentielles des compositions visées par la présente invention est de contenir au moins un pigment d'oxyde de titane. Les pigments visés par la présente invention sont les pigments d'oxyde de titane connus, habituellement utilisés dans le domaine cosmétique comme charges ou comme filtres, qui peuvent être traités ou non traités. De tels pigments comprennent les nanopigments d'oxyde de titane. Par nanopigments, on entend des pigments dont la taille moyenne des particules élémentaires est comprise entre 5 et 100 nm.

L'oxyde de titane peut se présenter sous forme rutile, anatase ou amorphe, mais de préférence sous forme rutile et/ou anatase.

Les pigments traités peuvent par exemple être traités par l'alumine, la silice, les composés de l'aluminium, les composés du silicium, les composés du sodium, les oxydes de fer, les esters de fer, l'acide stéarique, la glycérine.

Plus particulièrement, les pigments traités peuvent être des oxydes de titane traités par :

- la silice et l'alumine tels que les produits ⟨⟨ MICROTITANIUM DIOXIDE MT 500 SA ⟩⟩ et ⟨⟨ MICROTITANIUM DIOXIDE MT 100 SA ⟩⟩ de la société TAYCA, et les produits ⟨⟨ TIOVEIL Fin ⟩⟩, ⟨⟨ TIOVEIL OP ⟩⟩, ⟨⟨ TIOVEIL MOTG ⟩⟩ et ⟨⟨ TIOVEIL IPM ⟩⟩ de la société TIOXIDE,
- l'alumine et le stéarate d'aluminium tels que le produit ⟨⟨ MICROTITANIUM DIOXIDE MT 100 T ⟩⟩ de la société TAYCA,

- l'alumine et le laurate d'aluminium tels que le produit ⟨⟨ MICROTITANIUM DIOXIDE MT 100 S ⟩⟩ de la société TAYCA,
- des oxydes de fer et le stéarate de fer tels que le produit ⟨⟨ MICROTITANIUM DIOXIDE MT 100 F ⟩⟩ de la société TAYCA,
- la silice, l'alumine et la silicone tels que les produits ⟨⟨ MICROTITANIUM DIOXIDE MT 100 SAS ⟩⟩, ⟨⟨ MICROTITANIUM DIOXIDE MT 600 SAS ⟩⟩ et ⟨⟨ MICROTITANIUM DIOXIDE MT 500 SAS ⟩⟩ de la société TAYCA,
- l'hexamétaphosphate de sodium tels que le produit ⟨⟨ MICROTITANIUM DIOXIDE MT 150 W ⟩⟩ de la société TAYCA,
- l'octyltriméthoxysilane tels que le produit ⟨⟨ T-805 ⟩⟩ de la société DEGUSSA,
- l'alumine et l'acide stéarique tels que le produit ⟨⟨ UVT-M160 ⟩⟩ de la société KEMIRA,
- l'alumine et la glycérine tels que le produit ⟨⟨ UVT-M212 ⟩⟩ de la société KEMIRA,
- l'alumine et la silicone tels que le produit ⟨⟨ UVT-M262 ⟩⟩ de la société KEMIRA.

Les oxydes de titane non traités peuvent par exemple être ceux vendus par la société TAYCA sous les dénominations commerciales ⟨⟨ MICROTITANIUM DIOXIDE MT 500 B ⟩⟩ ou ⟨⟨ MICROTITANIUM DIOXIDE MT 600 B ⟩⟩.

Le ou les (nano)pigments d'oxyde de titane peuvent être présents dans la composition selon l'invention dans une proportion comprise entre 0,1 et 30% en poids par rapport au poids total de la composition, de préférence, entre 0,2 et 25% en poids par rapport au poids total de la composition.

Les autres constituants pouvant rentrer dans la formulation des compositions visées par l'invention, en particulier les huiles, les composés cireux, les épaississants, les émulsionnants, les gélifiants sont ceux qui sont classiquement utilisés dans le domaine cosmétique et/ou dermatologique.

Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (palmitate d'octyle, lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Comme émulsionnants, on peut utiliser les esters d'acides gras et de polyéthylène glycol (PEG), les esters d'acides gras et de glycérol (stéarate de glycéryle) ou les esters d'acides gras et de sucre (stéarate de sorbitane), ainsi que leurs dérivés polyoxyéthylénés ou polyoxypropylénés, les cyclométhicones et diméthicones copolyols, les tensioactifs anioniques (alkylphosphate de K ou de Na), les alcools gras polyalcoxylés.

De préférence, on utilise les alcools gras polyalcoxylés tels que les alcools butyliques oxypropylénés, les alcools capryliques oxyéthylénés, les alcools cétyliques oxyéthylénés.

Comme épaississants, on peut utiliser les acides polyacryliques réticulés, les gommes de guar et celluloses modifiées ou non telles que la gomme de guar hydroxypropylée, la méthylhydroxyéthylcellulose, l'hydroxypropylméthylcellulose ou encore l'hydroxyéthylcellulose.

Comme gélifiants, on peut citer les argiles modifiées (bentones), les sels métalliques d'acides gras (stéarate d'aluminium), les copolymères éthylène/acrylate, les silices, les polyéthylènes, les silicates de calcium ou encore l'éthylcellulose.

Les compositions visées par la présente invention peuvent aussi contenir divers ingrédients classiquement utilisés dans le domaine cosmétique, dermatologique ou dermopharmaceutique comme les matières colorantes, les solvants (eau, alcools,...), les conservateurs, les parfums, les actifs hydratants, les agents absorbant ou bloquant les rayons ultraviolets (filtres solaires organiques, pigments minéraux autres que les oxydes de titane, notamment les oxydes de cerium et/ou de zinc), des agents pulvérulents, des agents bactéricides et/ou des absorbeurs d'odeur.

Ces compositions peuvent, en outre, contenir un ou plusieurs actifs cosmétiques ou dermatologiques hydrophiles et mieux lipophiles, notamment en vue de traiter et/ou prévenir les affections cutanées telles que l'acné, les mycoses, l'eczéma, la rosacée, les dermites séborrhéïques, les hélio dermatoses, le vieillissement cutané ainsi que les affections du cuir chevelu. Ces compositions sont destinées au traitement de la peau par voie topique.

Bien entendu, l'homme de l'art veillera à choisir le ou les éventuels composés complémentaires cités ci-dessus et/ou leurs quantités de manière telle que les propriétés avantageuses attachées intrinsèquement à l'association binaire conforme à l'invention ne soient pas, ou substantiellement pas, altérées par la ou les adjonctions envisagées.

Les compositions concernées par l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement

être conditionnée en aérosol et se présenter sous forme de mousse ou de spray.

De préférence, cette composition se présente sous la forme d'une émulsion huile-dans-eau.

Des exemples concrets, mais nullement limitatifs, illustrant l'invention, vont maintenant être donnés.

**EXEMPLE 1:**

Des tests comparatifs ont été réalisés afin de mettre en évidence l'amélioration apportée au niveau du photobleuissement par l'introduction d'une silicone filtre conforme à l'invention au sein d'une composition contenant des pigments d'oxyde de titane.

On a ainsi réalisé trois émulsions huile-dans-eau A, B et C, chacune d'elles contenant un nanopigment de $TiO_2$ et une silicone benzotriazole. Les émulsions A et B, conformes à l'invention, contiennent respectivement le composé (a) selon l'invention et le composé (b) selon l'invention tels que définis ci-avant dans la description. L'émulsion C comparative contient une silicone benzotriazole non conforme à celles de l'invention (composé (c) décrit ci-dessous).

Ces trois émulsions sont de composition suivante (les quantités sont exprimées en poids, par rapport au poids total de la composition) :

| | |
|---|---|
| - silicone benzotriazole | 5 % |
| - mélange d'alcool cétylstéarylique et d'alcool cétylstéarylique oxyéthylèné à 33 OE (80/20) vendu sous la dénomination commerciale ⟨⟨ Sinnovax AO ⟩⟩ par Henkel | 7 % |
| - monostéarate de glycérol vendu sous la dénomination commerciale ⟨⟨ Géléol copeaux ⟩⟩ par Gattefossé | 2 % |
| - alcool cétylique vendu sous la dénomination commerciale ⟨⟨ Lorol C 16 ⟩⟩ par Henkel | 1,5 % |
| - benzoate d'alcool C12-C5 vendu sous la dénomination commerciale ⟨⟨ Finsolv TN ⟩⟩ par Finetex | 15 % |
| - polydiméthylsiloxane vendu sous la dénomination commerciale ⟨⟨ Silbione 70047 V 300 ⟩⟩ par Rhône-Poulenc | 1,5 % |
| - nanopigments de $TiO_2$ vendu sous la dénomination commerciale ⟨⟨ MT 100 T ⟩⟩ par Tayca | 5 % |
| - glycérine | 20 % |
| - conservateur | qs |
| - eau | qs 100 % |

Emulsion A : la silicone benzotriazole est le composé (a) de formule suivante :

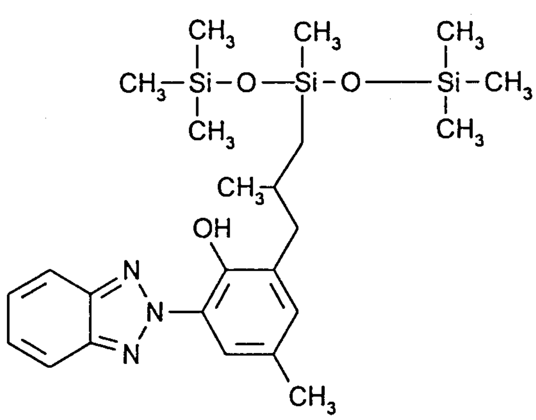

Emulsion B : la silicone benzotriazole est le composé (b) de formule suivante :

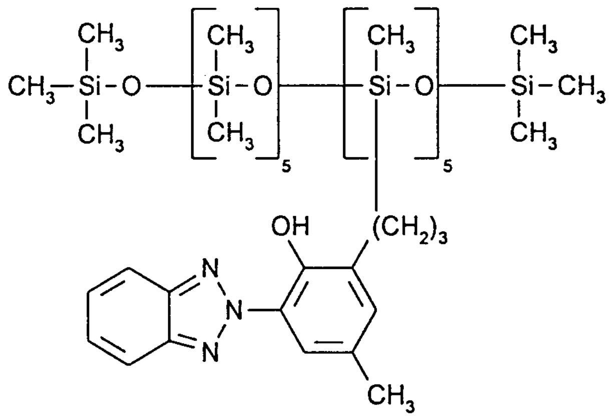

Emulsion C : la silicone benzotriazole est le composé (c) de formule suivante :

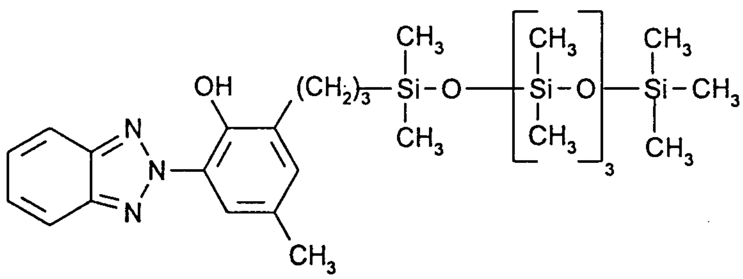

On a également réalisé une émulsion huile-dans-eau D de même composition que les émulsions A, B et C mais ne contenant aucune silicone benzotriazole.

Protocole d'évaluation du photobleuissement :

Pour ces quatre émulsions, le photobleuissement a été évalué selon le protocole suivant : les compositions ont été introduites dans des boîtes en plastique transparentes aux UV ( boîtes polystyrène cristal 50x40x6 $mm^3$) et exposées aux UV (SUNTEST CPS Heraeus) pendant 1H solaire. Les mesures colorimétriques ont été effectuées à l'aide d'un colorimètre Minolta CM1000 : une première mesure a été relevée juste avant l'exposition aux UV (T0) et une deuxième après une heure d'exposition aux UV (T1H).

Les résultats sont exprimés dans le système (L, a, b) dans lequel L représente la luminance, a représente l'axe rouge-vert (-a = vert, +a = rouge) et b représente l'axe jaune-bleu (-b =bleu, +b =jaune).

Pour l'évaluation du photobleuissement, on s'intéresse à :

1°) $\Delta L$ ($\Delta L = L_{T1H} - L_{T0}$) qui traduit l'assombrissement de la couleur : moins ΔL est négatif, moins la composition s'est assombrie,

2°) $\Delta b$ ($\Delta b = b_{T1H} - b_{T0}$) qui traduit le bleuissement de la couleur : moins Δb est négatif, plus la protection contre le photobleuissement est efficace.

Les résultats sont consignés dans le tableau (I) suivant :

Tableau (I)

| Emulsion | ΔL | Δb |
|---|---|---|
| Emulsion A (invention) | -11,5 | -9,7 |
| Emulsion B (invention) | -15,4 | -11,6 |

Tableau (I) (suite)

| Emulsion | ΔL | Δb |
|---|---|---|
| Emulsion C (comparatif) | -20,5 | -16,1 |
| Emulsion D (comparatif) | -33,1 | -22,0 |

Ces résultats montrent clairement que les émulsions contenant une silicone conforme à l'invention sont, de façon générale, très peu sujettes à l'assombrissement provoqué par une irradiation UV. Ils montrent également clairement que ces émulsions sont beaucoup moins sujettes au photobleuissement que des compositions ne contenant aucune silicone benzotriazole (émulsion D) ou contenant une autre silicone benzotriazole (émulsion C).

**Revendications**

**1.** Procédé pour réduire le photobleuissement d'une composition contenant des pigments d'oxyde de titane, caractérisé par le fait qu'il consiste à introduire dans ladite composition au moins une silicone benzotriazole répondant à l'une des formules suivantes :

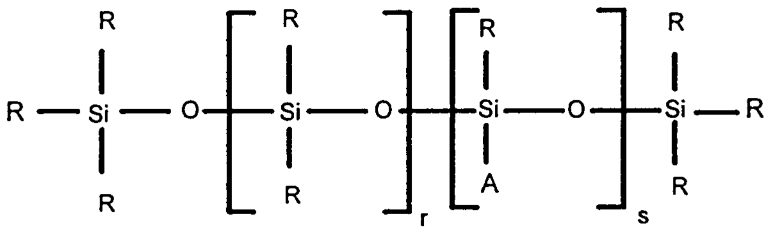

(1)

ou

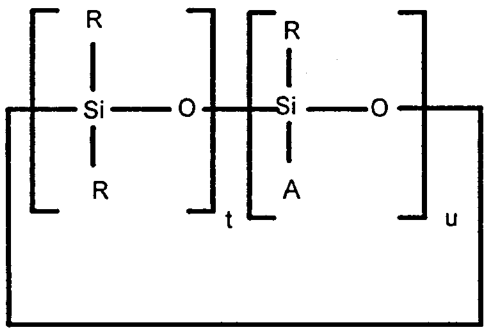

(2)

formules (1) et (2) dans lesquelles :

- R, identiques ou différents, sont choisis parmi les radicaux alkyles en $C_1$-$C_{10}$, phényle, trifluoro-3,3,3 propyle et triméthylsilyloxy, au moins 80% en nombre des radicaux R étant méthyle,
- r est un nombre entier compris entre 0 et 50 inclusivement, et s est un nombre entier compris entre 1 et 20 inclusivement,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

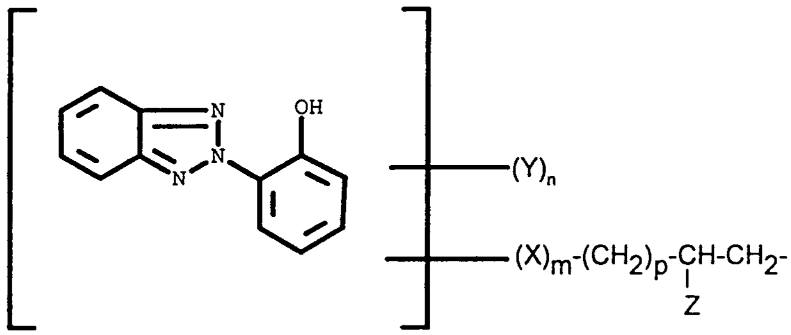

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

**2.** Procédé selon la revendication 1, caractérisé par le fait que la silicone benzotriazole est choisie parmi les composés de formule générale (1) présentant au moins l'une des caractéristiques suivantes :

- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

**3.** Procédé selon la revendication 2, caractérisé par le fait que ledit composé de formule générale (1) présente l'ensemble desdites caractéristiques.

**4.** Procédé selon la revendication 3, caractérisé par le fait que la silicone benzotriazole est choisie parmi les composés de formule générale (4) :

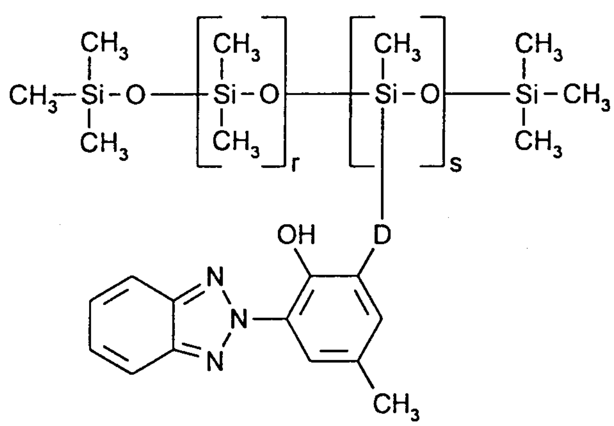

(4)

avec

$0 \leq r \leq 10$,
$1 \leq s \leq 10$ ,

et où D représente le radical divalent :

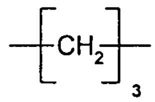

ou

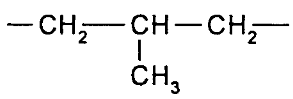

5. Procédé selon la revendication 4, caractérisé par le fait que la silicone benzotriazole répond à la formule générale (4) dans laquelle :

r=0
s=1
D=

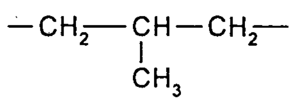

6. Procédé selon la revendication 4, caractérisé par le fait que la silicone benzotriazole répond à la formule générale (4) dans laquelle :

r = 5
s = 5

et D représente le radical divalent :

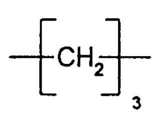

7. Procédé selon l'une quelconque des revendications précédentes, caractérisé par le fait que la silicone benzotriazole est présente dans ladite composition à une concentration comprise entre 0,1 et 15 % en poids par rapport au poids total de la composition.

8. Procédé selon la revendication 7, caractérisé par le fait que ladite concentration est comprise entre 0,2 et 10 % en poids par rapport au poids total de la composition.

9. Procédé selon l'une quelconque des revendications précédentes, caractérisé par le fait que les pigments d'oxyde

de titane présentent des particules élémentaires dont la taille moyenne est comprise entre 5 et 100 nm.

**10.** Procédé selon l'une quelconque des revendications précédentes, caractérisé par le fait que les pigments d'oxyde de titane sont présents dans la composition à une teneur allant de 0,1 à 30 % en poids par rapport au poids total de la composition.

**11.** Procédé selon la revendication 10, caractérisé par le fait que ladite teneur va de 0,2 à 25 % en poids, par rapport au poids total de la composition.

**12.** Utilisation d'au moins une silicone benzotriazole telle que définie à l'une quelconque des revendications 1 à 6 dans ou pour la fabrication d'une composition cosmétique et/ou dermatologique contenant des pigments d'oxyde de titane, pour diminuer le photobleuissement dû à la présence desdits pigments dans ladite composition.

**13.** Utilisation selon la revendication 12, caractérisée par le fait que la silicone benzotriazole est présente dans ladite composition à une concentration comprise entre 0,1 et 15 % en poids par rapport au poids total de la composition.

**14.** Utilisation selon la revendication 13, caractérisée par le fait que ladite concentration est comprise entre 0,2 et 10 % en poids par rapport au poids total de la composition.

**15.** Utilisation selon l'une quelconque des revendications 12 à 14, caractérisée par le fait que les pigments d'oxyde de titane présentent des particules élémentaires dont la taille moyenne est comprise entre 5 et 100 nm.

**16.** Utilisation selon l'une quelconque des revendications 12 à 15, caractérisée par le fait que les pigments d'oxyde de titane sont présents dans la composition à une teneur allant de 0,1 à 30 % en poids par rapport au poids total de la composition.

**17.** Utilisation selon la revendication 16, caractérisée par le fait que ladite teneur va de de 0,2 à 25 %.

**18.** Composition cosmétique et/ou dermatologique, en particulier pour la photoprotection de la peau et/ou des cheveux, caractérisée par le fait qu'elle comprend, dans un support cosmétiquement acceptable, des pigments d'oxyde de titane et au moins une silicone benzotriazole telle que définie à l'une quelconque des revendications 1 à 6, sous réserve que ladite composition est exempte d'acide benzène 1,4-di(3-méthylidène-10-camphosulfonique) et de 4,4'-méthoxy-t.-butyldibenzoylméthane.

**19.** Composition selon la revendication 18, caractérisée par le fait que la silicone benzotriazole est choisie parmi les composés de formule générale (1) présentant au moins l'une des caractéristiques suivantes :

- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

**20.** Composition selon la revendication 19, caractérisée par le fait que ledit composé de formule générale (1) présente l'ensemble desdites caractéristiques.

**21.** Composition selon la revendication 20, caractérisée par le fait que la silicone benzotriazole est choisie parmi les composés de formule générale (4) :

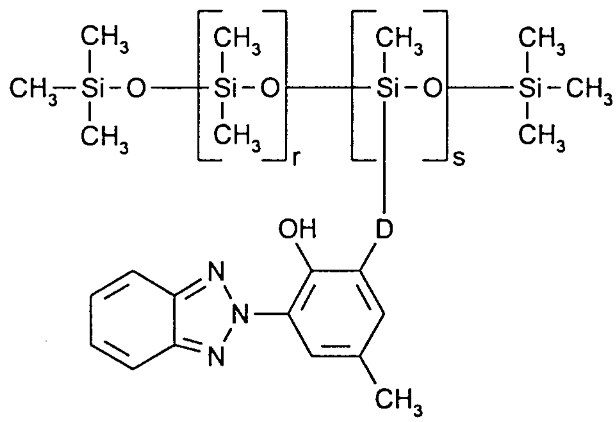

(4)

avec

0 ≤ r ≤ 10,
1 ≤ s ≤ 10 ,

et où D représente le radical divalent :

$$-[CH_2]_3-$$

ou

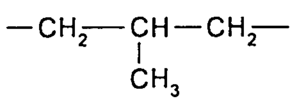

22. Composition selon la revendication 21, caractérisée par le fait que la silicone benzotriazole répond à la formule générale (4) dans laquelle :

r=0
s=1
D=

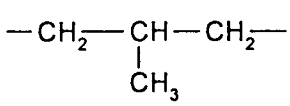

23. Composition selon la revendication 21, caractérisée par le fait que la silicone benzotriazole répond à la formule générale (4) dans laquelle :

r = 5
s = 5

et D représente le radical divalent :

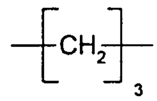

**24.** Composition selon l'une quelconque des revendications 18 à 23, caractérisée par le fait que la silicone benzotriazole est présente dans ladite composition à une concentration comprise entre 0,1 et 15 % en poids par rapport au poids total de la composition.

**25.** Composition selon la revendication 24, caractérisée par le fait que ladite concentration est comprise entre 0,2 et 10 % en poids par rapport au poids total de la composition.

**26.** Composition selon l'une quelconque des revendications 18 à 25, caractérisée par le fait que les pigments d'oxyde de titane présentent des particules élémentaires dont la taille moyenne est comprise entre 5 et 100 nm.

**27.** Composition selon l'une quelconque des revendications 18 à 26, caractérisée par le fait que les pigments d'oxyde de titane sont présents dans la composition à une teneur allant de 0,1 à 30 % en poids par rapport au poids total de la composition.

**28.** Composition selon la revendication 27, caractérisée par le fait que ladite teneur va de 0,2 à 25 %.

**29.** Procédé de traitement cosmétique pour protéger la peau et/ou les cheveux contre le rayonnement ultraviolet, en particulier le rayonnement solaire, caractérisé par le fait qu'il consiste à appliquer sur ceux-ci une quantité efficace d'au moins une composition définie à l'une des revendications 18 à 23.

## Claims

**1.** Process for reducing the photoblueing of a composition containing titanium oxide pigments, characterized in that it consists in introducing into the said composition at least one benzotriazole silicone corresponding to one of the following formulae:

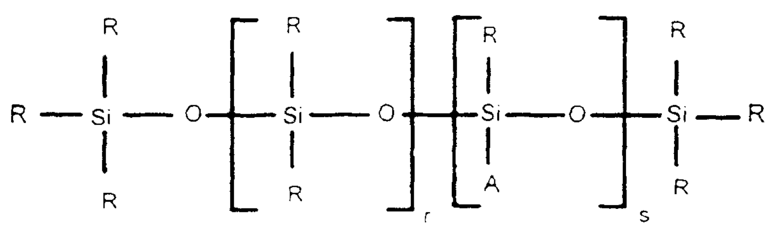

(1)

or

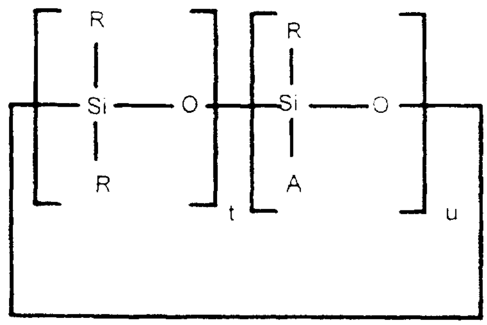

(2)

in which formulae (1) and (2):

- R, which are identical or different, are chosen from $C_1$-$C_{10}$ alkyl, phenyl, 3,3,3-trifluoropropyl and trimethylsilyloxy radicals, at least 80% by number of the R radicals being methyl,
- r is an integer of between 0 and 50 inclusive and s is an integer of between 1 and 20 inclusive,
- u is an integer of between 1 and 6 inclusive and t is an integer of between 0 and 10 inclusive, it being understood that t + u is equal to or greater than 3,
- and the symbol A denotes a monovalent radical bonded directly to a silicon atom which corresponds to the following formula (3):

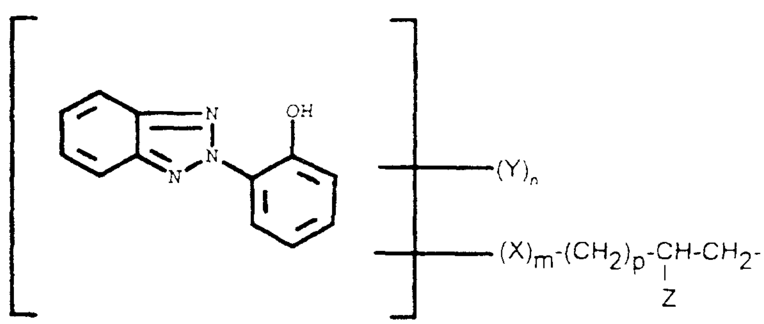

(3)

in which formula (3):

- Y, which are identical or different, are chosen from $C_1$-$C_8$ alkyl radicals, halogens and $C_1$-$C_4$ alkoxy radicals, it being understood that, in the latter case, two adjacent Y radicals of the same aromatic nucleus can together form an alkylidenedioxy group in which the alkylidene group contains from 1 to 2 carbon atoms,
- X represents O or NH,
- Z represents hydrogen or a $C_1$-$C_4$ alkyl radical,
- n is an integer of between 0 and 3 inclusive,
- m is 0 or 1,
- p represents an integer of between 1 and 10 inclusive.

2. Process according to Claim 1, characterized in that the benzotriazole silicone is chosen from the compounds of general formula (1) exhibiting at least one of the following characteristics:

- R is alkyl and more preferentially still is methyl,
- r is between 0 and 15 inclusive; s is between 1 and 10 inclusive,
- n is non-zero and preferably equal to 1 and Y is then chosen from methyl, tert-butyl or $C_1$-$C_4$ alkoxy,
- Z is hydrogen or methyl,
- m = 0 or [m = 1 and X = O ]

- p is equal to 1.

3. Process according to Claim 2, characterized in that the said compound of general formula (1) exhibits the combination of the said characteristics.

4. Process according to Claim 3, characterized in that the benzotriazole silicone is chosen from the compounds of general formula (4):

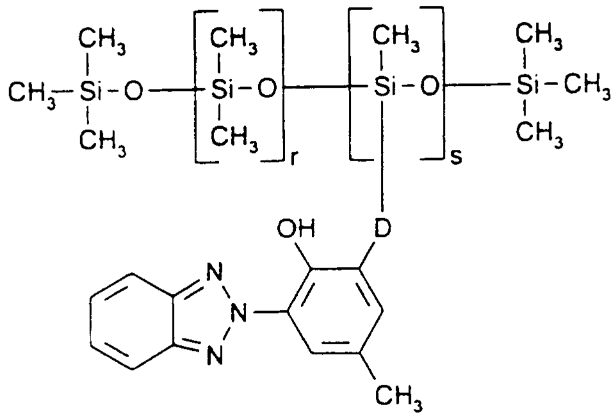

(4)

with

$0 \leq r \leq 10$,
$1 \leq s \leq 10$,

and where D represents the divalent radical:

$$-[CH_2]_3-$$

or

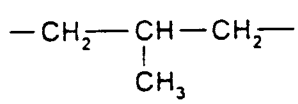

5. Process according to Claim 4, characterized in that the benzotriazole silicone corresponds to the general formula (4) in which:

r=0
s=1
D=

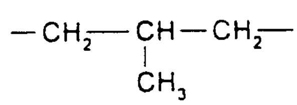

**6.** Process according to Claim 4, characterized in that the benzotriazole silicone corresponds to the general formula (4) in which:

r = 5
s = 5

and D represents the divalent radical:

$$-\left[CH_2\right]_3-$$

**7.** Process according to any one of the preceding claims, characterized in that the benzotriazole silicone is present in the said composition at a concentration of between 0.1 and 15% by weight with respect to the total weight of the composition.

**8.** Process according to Claim 7, characterized in that the said concentration is between 0.2 and 10% by weight with respect to the total weight of the composition.

**9.** Process according to any one of the preceding claims, characterized in that the titanium oxide pigments exhibit elementary particles with an average size of between 5 and 100 nm.

**10.** Process according to any one of the preceding claims, characterized in that the titanium oxide pigments are present in the composition at a content ranging from 0.1 to 30% by weight with respect to the total weight of the composition.

**11.** Process according to Claim 10, characterized in that the said content ranges from 0.2 to 25% by weight with respect to the total weight of the composition.

**12.** Use of at least one benzotriazole silicone as defined in any one of Claims 1 to 6 in or for the manufacture of a cosmetic and/or dermatological composition containing titanium oxide pigments for decreasing the photoblueing due to the presence of the said pigments in the said composition.

**13.** Use according to Claim 12, characterized in that the benzotriazole silicone is present in the said composition at a concentration of between 0.1 and 15% by weight with respect to the total weight of the composition.

**14.** Use according to Claim 13, characterized in that the said concentration is between 0.2 and 10% by weight with respect to the total weight of the composition.

**15.** Use according to any one of Claims 12 to 14, characterized in that the titanium oxide pigments exhibit elementary particles with an average size of between 5 and 100 nm.

**16.** Use according to any one of Claims 12 to 15, characterized in that the titanium oxide pigments are present in the composition at a content ranging from 0.1 to 30% by weight with respect to the total weight of the composition.

**17.** Use according to Claim 16, characterized in that the said content ranges from 0.2 to 25%.

**18.** Cosmetic and/or dermatological composition, in particular for the photoprotection of the skin and/or hair, characterized in that it comprises, in a cosmetically acceptable vehicle, titanium oxide pigments and at least one benzotriazole silicone as defined in any one of Claims 1 to 6, with the proviso that the said composition does not contain either benzene-1,4-di(3-methylidene-10-camphorsulphonic acid) or 4,4'-methoxy-t-butyldibenzoylmethane.

**19.** Composition according to Claim 18, characterized in that the benzotriazole silicone is chosen from the compounds of general formula (1) exhibiting at least one of the following characteristics:

- R is alkyl and more preferentially still is methyl,
- r is between 0 and 15 inclusive; s is between 1 and 10 inclusive,
- n is non-zero and preferably equal to 1 and Y is then chosen from methyl, tert-butyl or $C_1$-$C_4$ alkoxy,
- Z is hydrogen or methyl,
- m = 0 or [m = 1 and X = O]
- p is equal to 1.

**20.** Composition according to Claim 19, characterized in that the said compound of general formula (1) exhibits the combination of the said characteristics.

**21.** Composition according to Claim 20, characterized in that the benzotriazole silicone is chosen from the compounds of general formula (4):

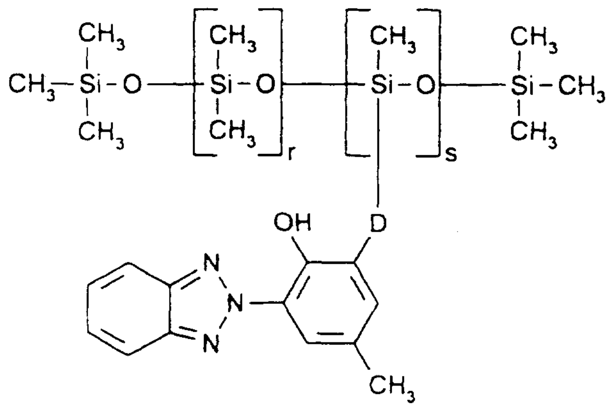

(4)

with

$0 \leq r \leq 10$,
$1 \leq s \leq 10$,

and where D represents the divalent radical:

$$-[CH_2]_3-$$

or

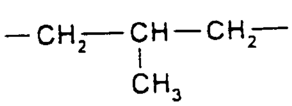

**22.** Composition according to Claim 21, characterized in that the benzotriazole silicone corresponds to the general formula (4) in which:

r=0
s=1
D=

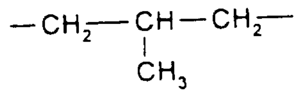

**23.** Composition according to Claim 21, characterized in that the benzotriazole silicone corresponds to the general formula (4) in which:

r = 5
s = 5

and D represents the divalent radical:

$$-\left[CH_2\right]_3-$$

**24.** Composition according to any one of Claims 18 to 23, characterized in that the benzotriazole silicone is present in the said composition at a concentration of between 0.1 and 15% by weight with respect to the total weight of the composition.

**25.** Composition according to Claim 24, characterized in that the said concentration is between 0.2 and 10% by weight with respect to the total weight of the composition.

**26.** Composition according to any one of Claims 18 to 25, characterized in that the titanium oxide pigments exhibit elementary particles with an average size of between 5 and 100 nm.

**27.** Composition according to any one of Claims 18 to 26, characterized in that the titanium oxide pigments are present in the composition at a content ranging from 0.1 to 30% by weight with respect to the total weight of the composition.

**28.** Composition according to Claim 27, characterized in that the said content ranges from 0.2 to 25%.

**29.** Cosmetic treatment process for protecting the skin and/or hair against ultraviolet radiation, in particular solar radiation, characterized in that it consists in applying on them an effective amount of at least one composition defined in one of Claims 18 to 23.

**Patentansprüche**

**1.** Verfahren zur Verminderung der durch Licht hervorgerufenen Blaufärbung von Zusammensetzungen, die Pigmente von Titandioxid enthalten, dadurch gekennzeichnet, daß es darin besteht, in diese Zusammensetzungen mindestens ein Benzotriazolsilicon einzubringen, das einer der folgenden Formeln entspricht:

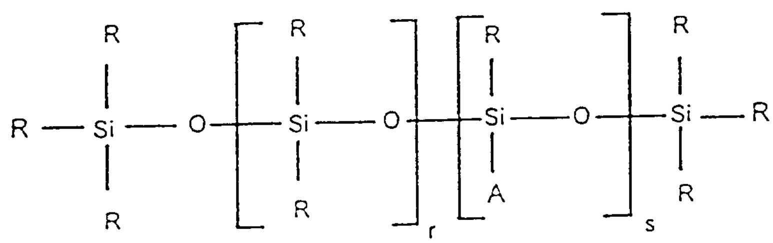 (1)

oder

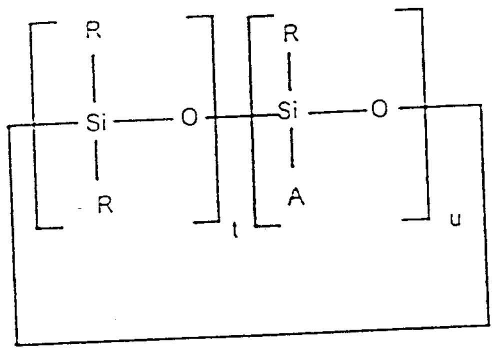

(2)

wobei in den Formeln (1) und (2):

- die Gruppen R, die identisch oder voneinander verschieden sind, unter $C_{1-10}$-Alkyl, Phenyl, 3,3,3-Trifluorpropyl und Trimethylsilyloxy ausgewählt sind, wobei es sich bei mindestens 80% der Anzahl der Gruppen R um Methyl handelt,
- r Null oder eine ganze Zahl im Bereich von 1 bis einschließlich 50 ist und s eine ganze Zahl im Bereich von 1 bis einschließlich 20 bedeutet,
- u eine ganze Zahl im Bereich von 1 bis einschließlich 6 ist und t Null oder eine ganze Zahl im Bereich von 1 bis einschließlich 10 bedeutet, mit der Maßgabe, daß t+u größer oder gleich 3 ist und
- das Symbol A eine einwertige Gruppe bezeichnet, die direkt an ein Siliciumatom gebunden ist und der folgenden Formel (3) entspricht:

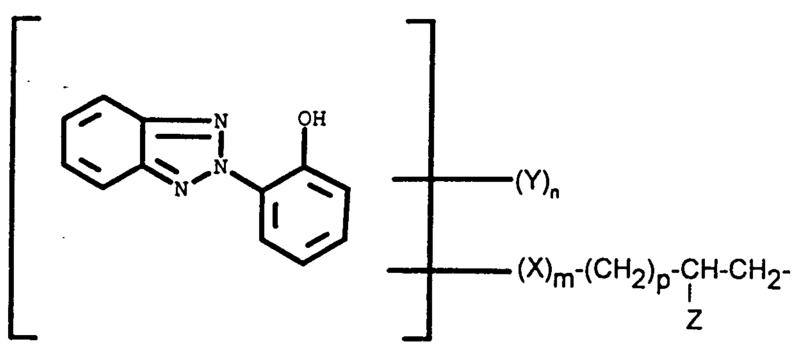

(3)

wobei in der Formel (3):

- die Gruppen Y, die identisch oder voneinander verschieden sind, unter $C_{1-8}$-Alkylgruppen, Halogenen und $C_{1-4}$-Alkoxygruppen ausgewählt sind, wobei in diesem letzten Fall zwei benachbarte Gruppen Y des gleichen aromatischen Rings zusammen eine Alkylidendioxygruppe bilden können, worin die Alkylidengruppe 1 bis 2 Kohlenstoffatome enthält,
- X O oder NH bedeutet,
- Z Wasserstoff oder eine $C_{1-4}$-Alkylgruppe bedeutet,
- n Null oder eine ganze Zahl im Bereich von 1 bis einschließlich 3 ist,
- m Null oder 1 ist und
- p eine ganze Zahl im Bereich von 1 bis einschließlich 10 bedeutet.

**2.** Verfahren nach Anspruch 1, dadurch gekennzeichnet, daß das Benzotriazolsilicon unter den Verbindungen der allgemeinen Formel (1) ausgewählt ist, die mindestens eine der folgenden Eigenschaften aufweisen:

- R bedeutet Alkyl und noch bevorzugter Methyl,
- r bedeutet Null oder einen Wert im Bereich von 1 bis einschließlich 15; s bedeutet einen Wert im Bereich von 1 bis einschließlich 10,
- n ist nicht Null und bedeutet vorzugsweise 1, und Y ist ausgewählt unter Methyl, *tert.*-Butyl oder $C_{1-4}$-Alkoxy,
- Z bedeutet Wasserstoff oder Methyl,
- m=0 oder [m=1 und X=O ] und
- p ist 1.

**3.** Verfahren nach Anspruch 2, dadurch gekennzeichnet, daß die Verbindung der allgemeinen Formel (1) sämtliche dieser Eigenschaften aufweist.

**4.** Verfahren nach Anspruch 3, dadurch gekennzeichnet, daß das Benzotriazolsilicon ausgewählt ist unter den Verbindungen der allgemeinen Formel (4):

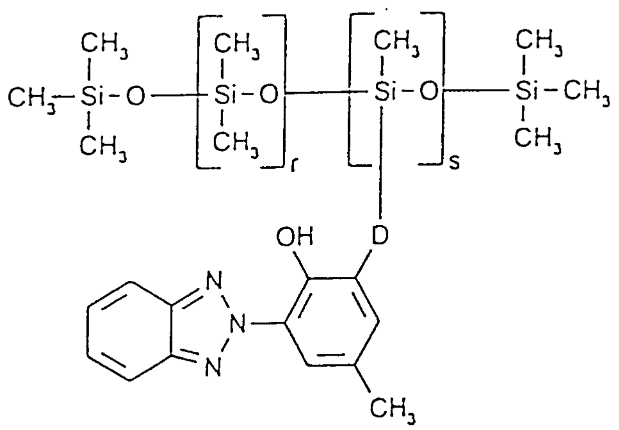

(4)

mit

$0 \leq r \leq 10,$
$1 \leq s \leq 10$

und worin D die zweiwertige Gruppe:

$$\left[-CH_2-\right]_3$$

oder

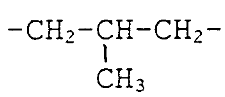

bedeutet.

**5.** Verfahren nach Anspruch 4, dadurch gekennzeichnet, daß das Benzotriazolsilicon der allgemeinen Formel (4) entspricht, worin:

r=0,
s=1,
D=

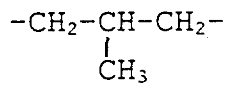
.

6. Verfahren nach Anspruch 4, dadurch gekennzeichnet, daß das Benzotriazolsilicon der allgemeinen Formel (4) entspricht, worin:

r=5,
s=5

und worin D die zweiwertige Gruppe :

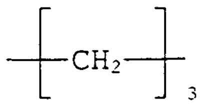

bedeutet.

7. Verfahren nach einem der vorhergehenden Ansprüche, dadurch gekennzeichnet, daß das Benzotriazolsilicon in der Zusammensetzung in einer Konzentration im Bereich von 0,1 bis 15 Gew.-%, bezogen auf das Gesamtgewicht der Zusammensetzung, vorliegt.

8. Verfahren nach Anspruch 7, dadurch gekennzeichnet, daß diese Konzentration im Bereich von 0,2 bis 10 Gew.-%, bezogen auf das Gesamtgewicht der Zusammensetzung, liegt.

9. Verfahren nach einem der vorhergehenden Ansprüche, dadurch gekennzeichnet, daß die Pigmente von Titandioxid Elementarpartikel mit einer mittleren Größe im Bereich von 5 bis 100 nm aufweisen.

10. Verfahren nach einem der vorhergehenden Ansprüche, dadurch gekennzeichnet, daß die Pigmente von Titandioxid in der Zusammensetzung in einem Mengenanteil von 0,1 bis 30 Gew.-%, bezogen auf das Gesamtgewicht der Zusammensetzung, vorliegen.

11. Verfahren nach Anspruch 10, dadurch gekennzeichnet, daß dieser Mengenanteil im Bereich von 0,2 bis 25 Gew.-%, bezogen auf das Gesamtgewicht der Zusammensetzung, liegt.

12. Verwendung mindestens eines Benzotriazolsilicons nach einem der Ansprüche 1 bis 6 in oder zur Herstellung von kosmetischen und/oder dermatologischen Zusammensetzungen, die Pigmente von Titandioxid enthalten, zur Verminderung der Photobläuung, die von der Anwesenheit dieser Pigmente in den Zusammensetzungen rührt.

13. Verwendung nach Anspruch 12, dadurch gekennzeichnet, daß das Benzotriazolsilicon in der Zusammensetzung in einer Konzentration im Bereich von 0,1 bis 15 Gew.-%, bezogen auf das Gesamtgewicht der Zusammensetzung, vorliegt.

14. Verwendung nach Anspruch 13, dadurch gekennzeichnet, daß diese Konzentration im Bereich von 0,2 bis 10 Gew.-%, bezogen auf das Gesamtgewicht der Zusammensetzung, liegt.

15. Verwendung nach einem der Ansprüche 12 bis 14, dadurch gekennzeichnet, daß die Pigmente von Titandioxid Elementarpartikel mit einer mittleren Größe im Bereich von 5 bis 100 nm aufweisen.

16. Verwendung nach einem der Ansprüche 12 bis 15, dadurch gekennzeichnet, daß die Pigmente von Titandioxid in der Zusammensetzung in einem Mengenanteil von 0,1 bis 30 Gew.-%, bezogen auf das Gesamtgewicht der Zusammensetzung, vorliegen.

**17.** Verwendung nach Anspruch 16, dadurch gekennzeichnet, daß dieser Mengenanteil im Bereich von 0,2 bis 25 % liegt.

**18.** Kosmetische und/oder dermatologische Zusammensetzungen, insbesondere zum Schutz der Haut und/oder der Haare gegenüber Licht, dadurch gekennzeichnet, daß sie in einem kosmetisch akzeptablen Träger Titandioxidpigmente und mindestens ein Benzotriazolsilicon nach einem der Ansprüche 1 bis 6 enthalten, mit der Maßgabe, daß diese Zusammensetzungen keine Benzol-1,4-di(3-methylidencampher-10-sulfonsäure) und kein 4,4'-Methoxy-*tert*.-butyldibenzoylmethan enthalten.

**19.** Zusammensetzungen nach Anspruch 18, dadurch gekennzeichnet, daß das Benzotriazolsilicon unter den Verbindungen der allgemeinen Formel (1) ausgewählt ist, die mindestens eine der folgenden Eigenschaften aufweisen:

- R bedeutet Alkyl und noch bevorzugter Methyl,
- r bedeutet Null oder einen Wert im Bereich von 1 bis einschließlich 15; s bedeutet einen Wert im Bereich von 1 bis einschließlich 10,
- n ist nicht Null und bedeutet vorzugsweise 1, und Y ist ausgewählt unter Methyl, *tert*.-Butyl oder $C_{1-4}$-Alkoxy,
- Z bedeutet Wasserstoff oder Methyl,
- m=0 oder [m=1 und X=O ] und
- p ist 1.

**20.** Zusammensetzungen nach Anspruch 19, dadurch gekennzeichnet, daß die Verbindung der allgemeinen Formel (1) sämtliche dieser Eigenschaften aufweist.

**21.** Zusammensetzungen nach Anspruch 20, dadurch gekennzeichnet, daß das Benzotriazolsilicon ausgewählt ist unter den Verbindungen der allgemeinen Formel (4):

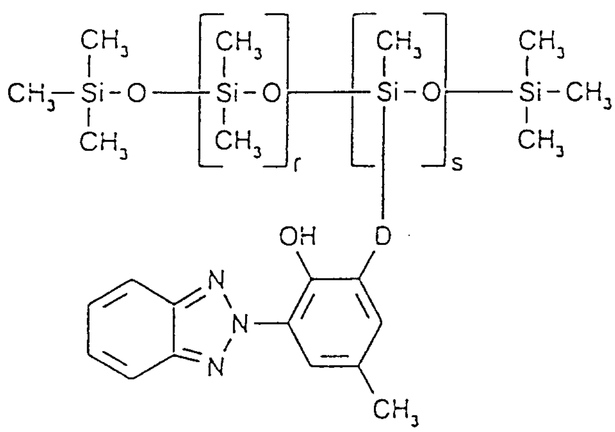

(4)

mit

$0 \leq r \leq 10$,
$1 \leq s \leq 10$

und worin D die zweiwertige Gruppe:

$$-\left[ CH_2 \right]_3-$$

oder

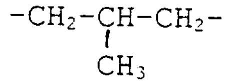

bedeutet.

**22.** Zusammensetzungen nach Anspruch 21, dadurch gekennzeichnet, daß das Benzotriazolsilicon der allgemeinen Formel (4) entspricht, worin:

r=0,
s=1,
D=

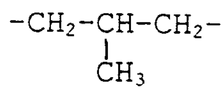

**23.** Zusammensetzungen nach Anspruch 21, dadurch gekennzeichnet, daß das Benzotriazolsilicon der allgemeinen Formel (4) entspricht, worin:

r=5,
s=5

und worin D die zweiwertige Gruppe:

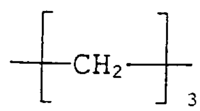

bedeutet.

**24.** Zusammensetzungen nach einem der Ansprüche 18 bis 23, dadurch gekennzeichnet, daß das Benzotriazolsilicon in diesen Zusammensetzungen in einer Konzentration im Bereich von 0,1 bis 15 Gew.-%, bezogen aus das Gesamtgewicht der Zusammensetzung, vorliegt.

**25.** Zusammensetzungen nach Anspruch 24, dadurch gekennzeichnet, daß diese Konzentration im Bereich von 0,2 bis 10 Gew.-%, bezogen auf das Gesamtgewicht der Zusammensetzung, liegt.

**26.** Zusammensetzungen nach einem der Ansprüche 18 bis 25, dadurch gekennzeichnet, daß die Pigmente von Titandioxid Elementarpartikel mit einer mittleren Größe im Bereich von 5 bis 100 nm aufweisen.

**27.** Zusammensetzungen nach einem der Ansprüche 18 bis 26, dadurch gekennzeichnet, daß die Pigmente von Titandioxid in den Zusammensetzungen in einem Mengenanteil von 0,1 bis 30 Gew.-%, bezogen auf das Gesamtgewicht der Zusammensetzung, vorliegen.

**28.** Zusammensetzungen nach Anspruch 27, dadurch gekennzeichnet, daß dieser Mengenanteil im Bereich von 0,2 bis 25 % liegt.

**29.** Verfahren zur kosmetischen Behandlung, um die Haut und/oder die Haare gegenüber ultravioletter Strahlung, insbesondere Sonnenstrahlung, zu schützen, dadurch gekennzeichnet, daß es darin besteht, auf die Haut und/oder die Haare eine wirksame Menge mindestens einer Zusammensetzung nach den Ansprüchen 18 bis 23 aufzutra-

gen.